# EUROPEAN PATENT SPECIFICATION

(11) **EP 3 162 349 B1**
(45) Date of publication and mention of the grant of the patent: **29.12.2021**
(21) Application number: 15812363.8
(22) Date of filing: 02.04.2015
(51) Int. Cl.: A61H 1/02, A61H 39/04, A61F 7/08, A61H 9/00

(54) **STRETCHING MASSAGE APPARATUS FOR SPINE CORRECTION AND MUSCLE STRENGTHENING**
STRECKMASSAGEVORRICHTUNG ZUR WIRBELSÄULENKORREKTUR UND MUSKELKRÄFTIGUNG
APPAREIL DE MASSAGE PAR ÉTIREMENT POUR LA CORRECTION DE LA COLONNE VERTÉBRALE ET LE RENFORCEMENT DES MUSCLES

(30) Priority: 26.06.2014 KR 20140004834 U
(43) Date of publication of application: 03.05.2017
(73) Proprietor: Kim, Han Il, Seoul 158-806 (KR)
(72) Inventor: BEAK, Eunseok, Seoul 122-755 (KR)
(74) Representative: Botti, Mario
(86) International application number: PCT/KR2015/003307
(87) International publication number: WO 2015/199321

(56) References cited:
- DE-C1- 19 716 268
- JP-A- 2010 051 597
- KR-A- 20020 082 445
- KR-A- 20050 040 696
- KR-B1- 101 257 732
- KR-B1- 101 398 677
- US-A- 5 637 076
- US-A1- 2007 283 496
- US-A1- 2010 139 003
- US-B1- 6 336 907
- None

## Description

### [Technical Field]

The present invention relates to a stretching massage apparatus for spine correction and muscle strengthening, and more particularly, to a human body stretching massage apparatus for a stretching massage of a human body.

### [Background Art]

Generally, a massage apparatus is an apparatus in which vibration generated by an operation of a motor is transmitted to a human body in contact with the massage apparatus and a massage for muscle relaxation and fatigue recovery of a corresponding part is performed.

Here, the massage is to promote metabolism by stimulating a main human meridian system and has a very long history as a physical therapy including acupuncture. Accordingly, the massage has been used as a folk remedy, but an effect thereof is recently recognized even in modern medicine. Further, even in the fields of sports, the massage is well used as a means for recovering from muscle fatigue to adjust athletes' physical condition.

Meanwhile, according to recent significant developments of electronics and machine-related industry fields, a lot of electronic therapeutic apparatuses have been launched. Also, various kinds of massage apparatuses have been developed from apparatuses for massaging limited body parts, e.g., feet or shoulder areas to apparatuses for massaging a whole body.

However, most of conventional massage apparatuses have protrusion type acupressure means for stimulating the main human meridian system and promoting the metabolism, as described above and thus have also very complicated massage operation structures.

Therefore, most of the conventional massage apparatuses just have structures for massaging or applying acupressure to each part of the human body but do not have a function of stretching and elongating each part of the human body, and each of persons should stretch his/her own body while taking a pose having an effect for stretching the human body to elongate the human body. Therefore, a development of a massage apparatus having the effect for stretching the human body is required.

Also, since the conventional massage apparatuses have the complicated structure for realizing a process of massaging or applying acupressure to each part of the human body, there are some problems that a manufacturing cost is increased and thus the massage apparatuses have no choice but to be provided to consumers at a high price.

Patent Document DE19716268 discloses a device comprising one or more inflatable cushions (a,b) not attached to the patient's body, connected by pipes to a controlled pressure supply to support the joint. Pressure in the cushions is increased and reduced with adjustable amplitude and frequency, so as to lift and lower the joint area without causing pain. The flexible expanding cushions can be made of materials with different coefficients of expansion as desired, several of them being connected via separate pipes to one or more pressure sources, their functioning being programme-controlled. The connections between the cushions can be movable.

Accordingly, the inventor of the present invention has developed a human body stretching massage apparatus which can solve the conventional problems, can elongate and stretch the human body while a user lies down comfortably without specific motions, can additionally allow acupressure and hot compress functions and thus can increase a massage effect on the human body.

### [Disclosure]

### [Technical Problem]

The present invention is directed to providing a human body stretching massage apparatus for a stretching massage of a human body.

### [Technical Solution]

One aspect of the present invention provides a human body stretching massage apparatus including a mattress including a plurality of expansion regions filled with a filler for expanding the mattress; a filler feeder connected to each of the expansion regions through a connection member to inject the filler into the expansion regions; and a controller configured to control an operation of the filler feeder, wherein the plurality of expansion regions are arranged in a lengthwise direction of the mattress, and the filler feeder may be formed to selectively supply the filler into the plurality of expansion regions.

The mattress may include an outer cover and a plurality of expansion members for forming the expansion regions. Each of the plurality of expansion members may have a length extending in a direction vertical to a lengthwise direction of the outer cover and the plurality of expansion members may be arranged in the lengthwise direction of the outer cover. The plurality of expansion members may be partially overlapped with each other.

Each of the plurality of expansion members may have a length which is equal to or smaller than a half of a width of the outer cover and the plurality of expansion members may be arranged in the lengthwise direction of the outer cover and a direction vertical to the lengthwise direction thereof.

Each of the plurality of expansion members may be disposed to be spaced apart from each other at a predetermined distance. Each of the expansion members may form at least one dividing line and may be divided into a plurality of regions.

The mattress may be formed by stacking two sheets and an edge of each of the sheets is sewed, and the expansion regions may divide the two sheets in a lengthwise direction thereof.

The apparatus may further include an acupressure protrusion installed inside the mattress.

In a descriptive example, the apparatus may further include a hot wire installed inside the mattress, and a hot wire power supply for supplying electric power to the hot wire and heating the hot wire.

The filler feeder may include a plurality of electronic valves which open and close the connection members connected to the plurality of expansion regions, and the controller may be configured to control opening and closing of the plurality of electronic valves.

### [Advantageous Effects]

In the human body stretching massage apparatus according to one embodiment of the present invention, it is possible to elongate and stretch the human body while a user lies down comfortably without specific motions, to additionally allow acupressure functions and thus to increase a massage effect on the human body.

### [Description of Drawings]

FIG. 1 is a view illustrating a configuration of a human body stretching massage apparatus according to one embodiment of the present invention.
FIG. 2 is a view illustrating a mattress of FIG. 1.
FIG. 3 is a view illustrating an expansion member of FIG. 1.
FIG. 4 is a view illustrating a human body stretching massage apparatus according to another embodiment of the present invention.
FIG. 5 is a view illustrating a human body stretching massage apparatus according to still another embodiment of the present invention.
FIGS. 6a to 6c are views illustrating a human body stretching massage apparatus according to yet another embodiment of the present invention.
FIG. 7 is a view illustrating a human body stretching massage apparatus according to still yet another embodiment of the present invention.
FIG. 8 is a view illustrating a human body stretching massage apparatus according to still yet another embodiment of the present invention.
FIG. 9 is a view illustrating a human body stretching massage apparatus according to still yet another embodiment of the present invention.
FIG. 10 is a view illustrating a human body stretching massage apparatus according to still yet another embodiment of the present invention.

### [Modes of the Invention]

A human body stretching massage apparatus according to the present invention includes a mattress including a plurality of expansion regions filled with a filler for expanding the mattress; a filler feeder connected to each of the expansion regions through a connection member to inject the filler into the expansion regions; and a controller configured to control an operation of the filler feeder, wherein the plurality of expansion regions are arranged in a lengthwise direction of the mattress, and the filler feeder may be formed to selectively supply the filler into the plurality of expansion regions.

Hereinafter, a human body stretching massage apparatus according to exemplary embodiments of the present invention will be described in detail with reference to the accompanying drawings. Although the present invention can be modified variously and have several embodiments, specific exemplary embodiments are illustrated in the accompanying drawings and will be described in detail in the detailed description. The same components are designated by the same reference numerals throughout the specification. In the accompanying drawings, dimensions of structures are shown on a larger scale than actual size for clarity of the present invention.

It will be understood that, although the terms first, second, etc. may be used herein to describe various elements, these elements should not be limited by these terms. These terms are only used to distinguish one element from another. For example, a first element could be termed a second element, and, similarly, a second element could be termed a first element, without departing from the scope of the present invention.

The terminology used herein is for the purpose of describing particular embodiments only and is not intended to be limiting of the invention. As used herein, the singular forms "a," "an" and "the" are intended to include the plural forms as well, unless the context clearly indicates otherwise. It will be further understood that the terms "comprises," "comprising," "includes" and/or "including," when used herein, specify the presence of stated features, integers, steps, operations, elements, components and/or a combination thereof, but do not preclude the presence or addition of one or more other features, integers, steps, operations, elements, components and/or a combination thereof.

Unless otherwise defined, all terms including technical and scientific terms used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined here.

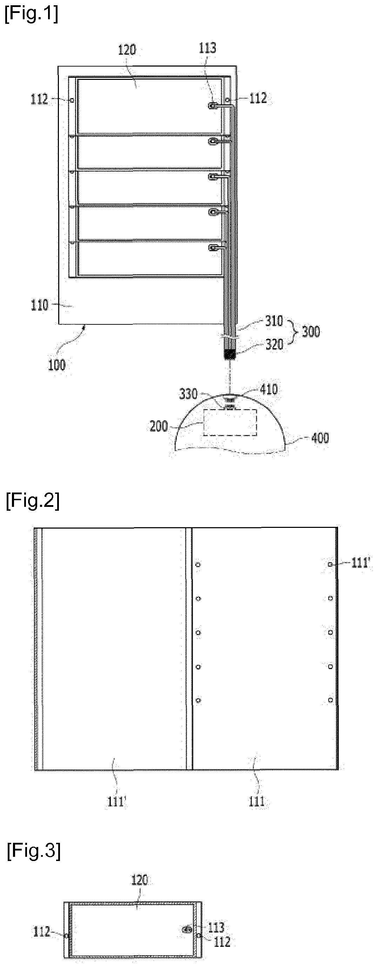

FIG. 1 is a view illustrating a configuration of a human body stretching massage apparatus according to one embodiment of the present invention, FIG. 2 is a view illustrating a mattress of FIG. 1, and FIG. 3 is a view illustrating an expansion member of FIG. 1.

Referring to FIGS. 1 and 2, the human body stretching massage apparatus according to one embodiment of the present invention includes a mattress 100, a filler feeder 200 and a controller 400.

The mattress 100 is a configuration for partially expanding and assisting stretching of a human body. The mattress 100 includes a plurality of expansion regions which are filled with a filler to allow the mattress 100 to expand. The mattress 100 may include an outer cover 110 and a plurality of expansion members 120.

The outer cover 110 forms an outer surface of the mattress 100. For example, the outer cover 110 may be formed of a synthetic resin having elasticity, and a cushion material (not shown) may be provided inside the outer cover 110. The outer cover 110 may be formed in, for example, a rectangular shape having a long length which may support the human body from a neck area to a waist area.

For example, the outer cover 110 may be configured with a plurality of sheets. In this case, male and female Velcro tapes may be installed at an edge of each of the sheets, and the sheets may be attached to each other by the male and female Velcro tapes and may form the outer cover 110. When the cushion material is provided at the outer cover 110, the cushion material may be provided to be stacked on an inner surface of one of the plurality of sheets.

The plurality of expansion members 120 are located in the plurality of expansion regions. For example, each of the expansion members 120 may be formed in a tube shape forming an accommodation space which may accommodate the filler. The plurality of expansion members 120 may be arranged in a lengthwise direction of the outer cover 110, for example, in a rectangular shape. At this point, the rectangular shape may have a length which extends in a direction vertical to the lengthwise direction of the outer cover. Also, the plurality of expansion members 120 may be configured to be partially overlapped with each other when being arranged in the lengthwise direction of the outer cover 110. Alternatively, the plurality of expansion members 120 may be disposed to be spaced apart from each other in a predetermined distance when being arranged in the lengthwise direction of the outer cover 110. The plurality of expansion members 120 may expand when the filler is injected therein.

The plurality of expansion members 120 may be fixed to an inner surface of the outer cover 110. For example, each of the plurality of expansion members 120 may include a female button 112, and a male button 112' may be provided at the inner surface of the outer cover 110. The plurality of expansion members 120 may be fixed to the inside of the outer cover 110 by coupling between the female button 112 and the male button 112'. Also, the plurality of expansion members 120 may form a filler inlet 113 through which the filler is introduced.

Otherwise, when the cushion material is provided inside the outer cover 110, the male button 112' may be provided on the cushion material. In this case, the plurality of expansion members 120 may be installed on the cushion material.

For example, 5 expansion members 120 may be provided. In this case, when a user lies down on the mattress 100, the first expansion member 120 may be located at the neck area of the human body, the second expansion member 120 may be located at the shoulder area thereof, the third expansion member 120 may be located at the shoulder area and the back area thereof, the fourth expansion member 120 may be located at the back area thereof and the fifth expansion member 120 may be located at the waist area thereof.

The filler feeder 200 is connected to an expansion region, i.e., each of the expansion members 120 through a connection member 300 and injects the filler into the expansion members 120. For example, the connection member 300 may include a plurality of hoses 310 having the number corresponding to the number of the expansion members 120 and a connector 320 connected to each of the hoses 310, and the filler may be air. In this case, the filler feeder 200 is a configuration for supplying the filler and may be a pneumatic device for supplying and exhausting the air. Also, the filler feeder 200 may include a plurality of electronic valves 330. The plurality of electronic valves 330 may be installed on the connection member 300. For example, the plurality of electronic valves 330 may be located at one end of the connection member 300 fixed to the expansion member 120 or the other end of the connection member 300 fixed to the filler feeder 200. The plurality of electronic valves 330 are electrically connected to the controller 400.

The controller 400 controls the filler feeder 200. For example, the controller 400 may include a casing, a control circuit configured with a circuit to control the filler feeder 200, a power supply and operation buttons. The control circuit may include a timer which counts an injection time to sequentially inject the filler into the plurality of expansion members 120 for a predetermined time in arrangement order of the plurality of expansion members 120. The operation buttons may include a power button for driving the filler feeder 200, a timer setting button, a selection button for selecting one or more of the plurality of expansion members 120 and so on. Also, the casing of the controller 400 may include a portion 410 to which the connector 320 of the above-mentioned connection member 300 is coupled. The filler feeder 200 may be installed inside the controller 400 and may be connected to the connector 320.

Hereinafter, a using method of the human body stretching massage apparatus according to one embodiment of the present invention will be described.

For a stretching massage of the human body, first, the user lies down on the mattress 100 while the mattress 100 is put on a floor. At this point, the mattress 100 is located to support the user's upper body.

Then, the user may push, for example, the power button of the driving buttons of the controller 400 and then may push the selection button. At this point, the selection button may be a button which sets the plurality of expansion members 120 to expand in turn for a predetermined time. In this case, the controller 400 may control the filler feeder 200 through the control circuit and may expand the expansion members 120 for a predetermined time. At this point, the time for which the plurality of expansion members 120 expand in turn may be counted by the timer.

For example, when 5 expansion members 120 may be provided and an expansion time of each of the expansion members 120 is 3 minutes, first, the air is supplied to the first expansion member 120, and an expanding state is maintained for 3 minutes to lift up and elongate the neck area of the human body, and then the air is exhausted from the expansion members 120 after 3minutes.

Then, the air is supplied to the second expansion member 120, and the expanding state is maintained for 3 minutes to lift up and elongate the shoulder area of the human body, and then the air is exhausted from the expansion members 120 after 3 minutes.

Then, the air is supplied to the third expansion member 120, and the expanding state is maintained for 3 minutes to lift up and elongate the shoulder area and the back area of the human body, and then the air is exhausted from the expansion members 120 after 3 minutes.

Then, the air is supplied to the fourth expansion member 120, and the expanding state is maintained for 3 minutes to lift up and elongate the back area of the human body, and then the air is exhausted from the expansion members 120 after 3 minutes.

Finally, the air is supplied to the fifth expansion member 120, and the expanding state is maintained for 3 minutes to lift up and elongate the waist area of the human body, and then the air is exhausted from the expansion members 120 after 3 minutes.

Such a stretching massage process of the human body is just an example and is not specifically limited thereto. Unlike the example, the expansion time of each of the expansion members 120 may become longer or shorter. When the expansion time of each of the expansion members 120 becomes longer, a stretching time of each area of the upper human body may be increased, and when the expansion time of each of the expansion members 120 becomes shorter, the expansion members 120 may repeatedly and rapidly elongate and stretch each area of the upper human body while repeatedly rapidly performing expansion and contraction at a high speed.

Since the human body stretching massage apparatus according to one embodiment of the present invention elongates and stretches each area of the human body by the repeated expansion and contraction of the expansion members 120 while the user lies on the mattress 100, the user may perform the stretching while comfortably lying down, and thus the stretching may be performed without the user's specific motions.

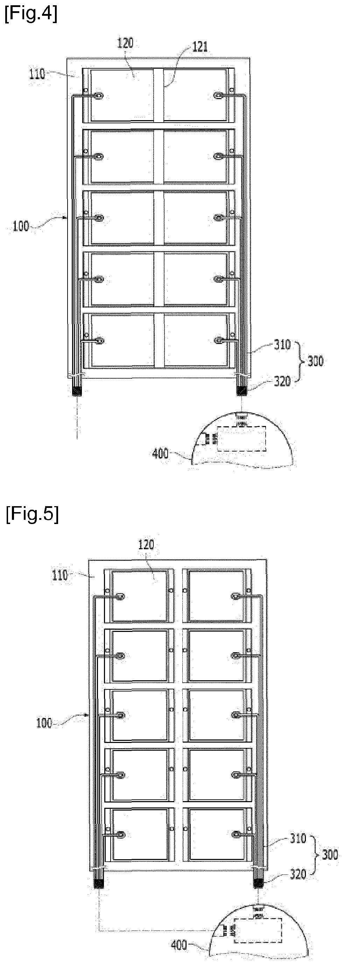

Hereinafter, a human body stretching massage apparatus according to another embodiment of the present invention will be described in detail with reference to FIG. 4, focusing on a difference from the human body stretching massage apparatus according to one embodiment of the present invention. FIG. 4 is a view illustrating a human body stretching massage apparatus according to another embodiment of the present invention.

Referring to FIG. 4, the human body stretching massage apparatus according to another embodiment of the present invention is the same as the human body stretching massage apparatus according to one embodiment of the present invention, except that each of the expansion members 120 forms at least one dividing line 121 and is divided into a plurality of regions.

For example, each of the expansion members 120 may form two dividing lines 121 in a direction vertical to a lengthwise direction of the expansion member 120 and may be divided into two regions. In this case, each of the divided regions may be filled with the filler. To this end, the connection member 300 may be connected to each of the divided regions, and the electronic valve 330 may be installed at the connection member 300. Also, the control circuit may control the filler feeder 200 so that the filler is injected into each of the divided regions.

In the human body stretching massage apparatus according to another embodiment of the present invention, since each of the divided regions in each of the expansion members 120 may be filled with the filler and may expand separately, not only a function of lifting up and elongating each area of the human body but also a function of twisting and stretching the human body may be included.

For example, when a region located at a left or right side of each of the expansion members 120 based on the dividing line 121 is filled with the filler and expands, the human body, e.g., a left or right portion of the upper body may be lifted up and stretched toward a right or left side.

As another example, when the air is injected into regions of some of the plurality of expansion members 120 which are located at the left side based on the dividing line and is also injected into regions of the remaining expansion members 120 which are located at the right side based on the dividing line, a part of the human body, e.g., the upper body is twisted and stretched to the left side, and another part thereof is twisted and stretched to the right side.

Therefore, the human body stretching massage apparatus according to another embodiment of the present invention may increase a human body stretching effect.

Hereinafter, a human body stretching massage apparatus according to still another embodiment of the present invention will be described in detail with reference to FIG. 5, focusing on a difference from the human body stretching massage apparatus according to one embodiment of the present invention. FIG. 5 is a view illustrating a human body stretching massage apparatus according to still another embodiment of the present invention.

Referring to FIG. 5, the human body stretching massage apparatus according to still another embodiment of the present invention is the same as the human body stretching massage apparatus according to one embodiment of the present invention, except that each of the expansion members 120 has a length which is equal to or smaller than a half of a width of the outer cover 110, is arranged in the lengthwise direction of the outer cover 110 and a direction vertical to the lengthwise direction thereof and disposed to be spaced part from each other.

^{∗} The human body stretching massage apparatus according to still another embodiment of the present invention has the same functions and effects as those in the human body stretching massage apparatus according to another embodiment of the present invention which has been described with reference to FIG. 4.

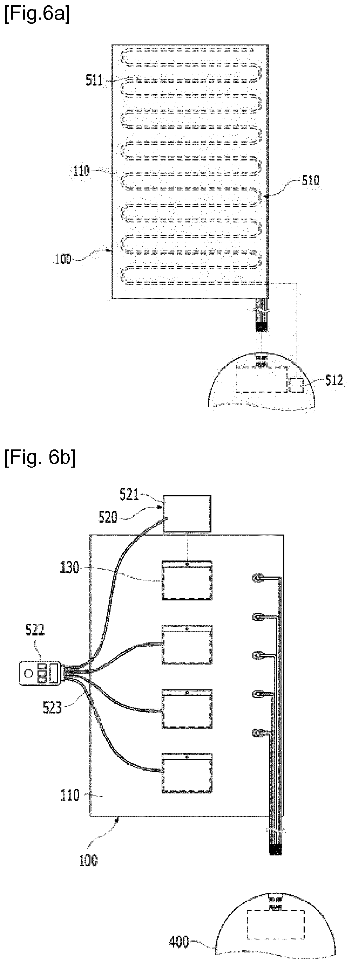

^{∗} Hereinafter, a human body stretching massage apparatus according to yet another embodiment of the present invention will be described in detail with reference to FIGS. 6a to 6c, focusing on a difference from the human body stretching massage apparatus according to one embodiment of the present invention. FIGS. 6a to 6c are views illustrating a human body stretching massage apparatus according to yet another embodiment of the present invention.

^{∗} Referring to FIGS. 6a to 6c, the human body stretching massage apparatus according to yet another embodiment of the present invention is the same as the human body stretching massage apparatus according to one embodiment of the present invention, except that thermal devices 510, 520 and 530 which are installed at the mattress 100 and perform a hot compress function for a body part in contact with the mattress 100 are further included.

FIG. 6a illustrates an example of the thermal device. Referring to FIG. 6a, the thermal device 510 includes a hot wire 511 installed inside the mattress 100, and a hot wire power supply 512 for supplying electric power to the hot wire 511 and heating the hot wire.

For example, the hot wire 511 may be fixed to the inner surface of the outer cover 110 of the mattress 100, and the hot wire power supply 512 may be installed inside the controller 400.

In the case of such a thermal device 510, when the electric power is supplied to the hot wire 511 through the hot wire power supply 512 and thus the hot wire 511 is heated, the outer cover 110 of the mattress 100 has heat having a predetermined temperature. Accordingly, when the plurality of expansion members 120 perform the stretching massage by the expansion and contraction, the heat is applied to a contact area of the human body, and thus the hot compress function may be performed.

FIG. 6b illustrates another example of the thermal device. Referring to FIG. 6b, the thermal device 520 includes a hot pack 521 filled with a thermal material and a power supply 522 for supplying the electric power to the hot pack 521 and heating the thermal material. In this case, a plurality of accommodation holes 130 for accommodating the hot pack 521 may be formed at an outer surface of the mattress 100.

The hot pack 521 may be removably installed in each of the accommodation holes 130 and may be a usual pack, and a liquid fuel or a powder material which generates heat by a contact may be provided therein.

For example, a supersaturated solution such as sodium acetate or sodium thiosulfate may be used as a filler of the hot pack 521. When a solid in a hydrate state is put in a pack and then heated after applying a predetermined amount of water, the solid may be changed into the supersaturated solution and may generate the heat while being changed again into the solid by an external impact.

As another example, the hot pack 521 may employ a method in which a vinyl pack is filled with a fluid, piezoelectric ceramic is provided therein and the pack is heated by connecting a power supply wire 523 to the power supply 522.

The power supply 522 may supply the electric power to the hot pack 521. The power supply 522 may be configured to adjust a heating temperature of the hot pack 521 or the like.

The accommodation holes 130 may be configured to be openable and closeable by installing a button, a Velcro tape, a zipper or the like at an upper side thereof.

In the case of the thermal device 510, when the electric power is supplied to the hot pack 521 through the power supply 522, the filler in the hot pack 521 generates the heat, and thus the hot compress may be applied to the body part in contact with the mattress 100.

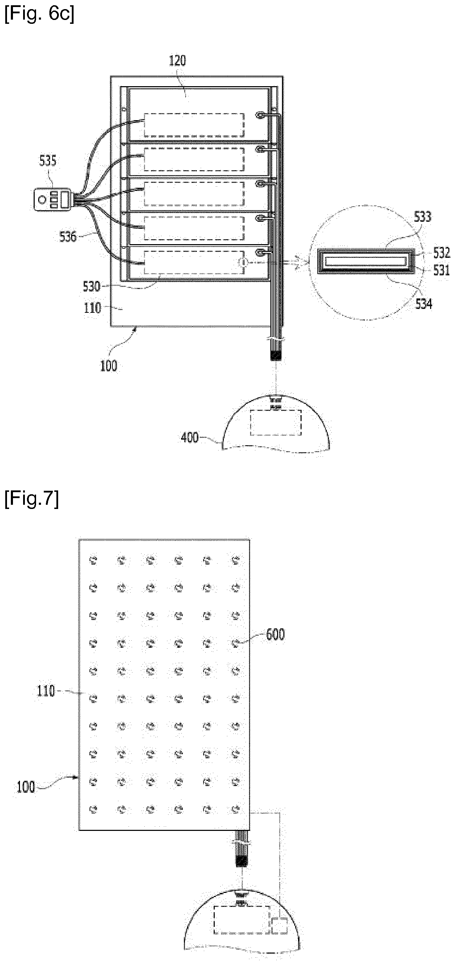

FIG. 6c illustrates still another example of the thermal device. Referring to FIG. 6c, the thermal device 530 is formed in a tube shape, and the tube may include a plate-shaped near-infrared radiator 531 which is formed of one or more selected from the group consisting of silica and alumina and emits near-infrared rays by heating; an exothermic body 532 which is formed of one or more selected from the group consisting of sodium, magnesium, aluminum, calcium, iron, zinc and manganese, coated on a surface of the near-infrared radiator and generates Joule heat; an electrode layer 533 which is formed of one or more selected from the group consisting of tungsten, tin and copper and formed at both ends of the exothermic body to connect a power source; and a protective layer 534 which is coated on a surface of the exothermic body to protect the exothermic body.

The near-infrared radiator 531 has a plate shape, is formed of a ceramic PTC (Positive Temperature Coefficient) exothermic material and coated with the exothermic body 532 which generates the Joule heat at a ceramic surface absorbing heat and emitting the Joule heat.

The nonmetallic PTC has excellent flexibility and durability and electrical stability, may be variously used within a range of 6 to 440V due to excellent thermal resistance and cold resistance and may generally perform a function at a temperature of 20 to 150°C.

In addition, since the nonmetallic PTC also has a brittle point of -60 to -70°C and thus still maintains elasticity even at a lower temperature at which a general organic compound is shocked or broken, it may be applied to a heating material for human body and the whole industry such as industry, agricultural and fishing industry, civil engineering industry and general home.

Both ends of the exothermic body 532 is coated with a metallic material, in which tungsten, tin and copper are mixed to ensure excellent conductivity for power supply and durability against thermal expansion, in a metallizing method.

The near-infrared radiator 531 is formed of a ceramic material including alumina of 81.9 weight%, silica of 8.1 weight%, carbon of 3.8 weight %, magnesium oxide of 1.25 weight% and other inorganic materials, and the ceramic material is machined in the plate shape and then processed by a plastic working.

In the case of the exothermic body 532, an outer circumferential surface of the near-infrared radiator 531 is sprayed and coated with sodium and magnesium solutions and then processed by the plastic working for 15 hours or more in a drying furnace maintained at a temperature of 1300°C.

The electrode layer 533 is formed of a conductive material including tungsten, tin and copper in the metallizing method.

And the protective layer 534 is coated on the exothermic body 532 except the both ends thereof to prevent oxidation of the exothermic body 532 and also to protect the exothermic body 532 from an external shock and then treated by heating in the drying furnace.

The tube-shaped thermal device 530 manufactured through such a process has a structure in which the exothermic body 532 as a resistor is coated on the plate-shaped near-infrared radiator 531 and the protective layer 534 is coated on the entire portion thereof except the both ends and the electrode layer 533 is coated on the both ends.

However, since the near-infrared radiator 531, the exothermic body 532 and the protective layer 534 and so on have thermal expansion coefficients different from each other, cracks may occur by the expansion and contraction, and thus when the exothermic body 532 and the protective layer 534 are processed into fine powder and then sprayed and coated, a chrome coating solution as a bonding agent of the powder may be added.

In the case of the thermal device 530, a power supply 535 separately provided is electrically connected by a power connection wire 536, the electric power is supplied from the power supply 535, the exothermic body 532 generates the heat, the mattress 100 is heated by the exothermic body 532, and thus the hot compress may be applied to the body part in contact with the mattress 100.

Also, since the thermal device 530 is formed in the tube shape and the tube is formed of an alumina-based inorganic material, it is strong against a thermal shock and light in weight and has excellent thermal conductivity, and the mattress 100 may be rapidly heated, and thus the hot compress function may be rapidly used.

Accordingly, when the human body stretching massage apparatus according to yet another embodiment of the present invention including the thermal devices 510, 520 and 530 is used, not only the stretching massage but also the hot compress may be enabled, and tense muscles of the human body may be rapidly relaxed, and thus the human body stretching effect may be further increased.

Hereinafter, a human body stretching massage apparatus according to still yet another embodiment of the present invention will be described in detail with reference to FIG. 7, focusing on a difference from the human body stretching massage apparatus according to one embodiment of the present invention. FIG. 7 is a view illustrating a human body stretching massage apparatus according to still yet another embodiment of the present invention.

Referring to FIG. 7, the human body stretching massage apparatus according to still yet another embodiment of the present invention is the same as the human body stretching massage apparatus according to one embodiment of the present invention, except that an acupressure protrusion 600 installed inside the mattress 100 is further included.

Here, a position at which the acupressure protrusion 600 is fixed is not specifically limited. For example, the acupressure protrusion 600 may be attached to the inner surface of the outer cover 110 of the mattress 100.

The human body stretching massage apparatus according to still yet another embodiment of the present invention additionally has a function in which the acupressure protrusion 600 presses a contact portion of the human body when the expansion members 120 expand while the stretching massage is performed by the expansion and contraction of the expansion members 120 and thus an acupressure therapy is applied to the human body.

Therefore, in the human body stretching massage apparatus according to still yet another embodiment of the present invention, the acupressure function with respect to the human body is provided together with the function of stretching each part of the human body, and thus a human body massage effect may be further increased.

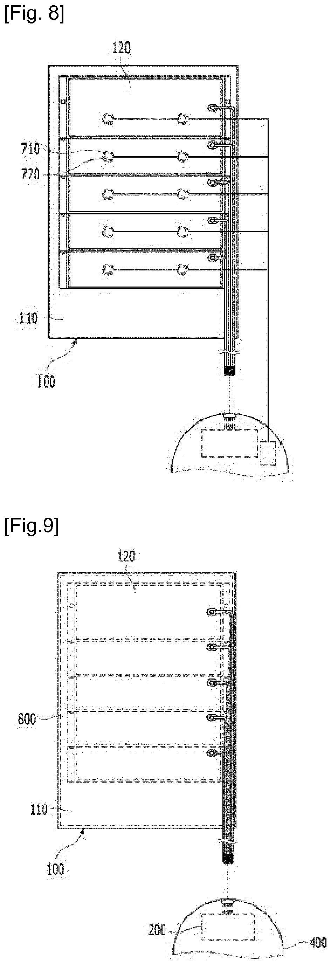

Hereinafter, a human body stretching massage apparatus according to still yet another embodiment of the present invention will be described in detail with reference to FIG. 8, focusing on a difference from the human body stretching massage apparatus according to one embodiment of the present invention. FIG. 8 is a view illustrating a human body stretching massage apparatus according to still yet another embodiment of the present invention.

Referring to FIG. 8, the human body stretching massage apparatus according to still yet another embodiment of the present invention is the same as the human body stretching massage apparatus according to the invention, except that an acupressure member 710 provided on a surface of the expansion member 120 and formed to have an accommodation space therein and a far-infrared lamp 720 accommodated in the accommodation space are further included.

A shape of the acupressure member 710 is not specifically limited. For example, the acupressure member 710 may be formed in a cylindrical shape of which a flat surface has a circular shape, as illustrated in FIG. 8. The cylinder may be hollow inside. A hollow inside of the cylinder may form an accommodation space which accommodates the far-infrared lamp 720.

The far-infrared lamp 720 emits far-infrared rays when emitting light. The far-infrared lamp 720 receives the electric power from a power supply inside the controller 400 and emits the light.

The human body stretching massage apparatus according to still yet another embodiment of the present invention additionally has a function in which the acupressure member 710 presses a contact portion of the human body when the expansion members 120 expand while the stretching massage is performed by the expansion and contraction of the expansion members 120 and thus the acupressure therapy is applied to the human body.

Also, since the acupressure member 710 accommodates the far-infrared lamp 720 therein, the far-infrared rays may be emitted and provided to the human body.

Therefore, in the human body stretching massage apparatus according to still yet another embodiment of the present invention, the acupressure function with respect to the human body is provided together with the function of stretching each part of the human body, and thus a human body massage effect may be further increased. Also, health of a user's body may be increased by emitting the far-infrared rays.

Hereinafter, a human body stretching massage apparatus according to still yet another embodiment of the present invention will be described in detail with reference to FIG. 9, focusing on a difference from the human body stretching massage apparatus according to one embodiment of the present invention. FIG. 9 is a view illustrating a human body stretching massage apparatus according to still yet another embodiment of the present invention.

Referring to FIG. 9, the human body stretching massage apparatus according to still yet another embodiment of the present invention is the same as the human body stretching massage apparatus according to one embodiment of the present invention, except that a support plate 800 installed under the mattress 100 or inside the mattress 100 to support the expansion members 120 when the expansion members 120 expand is further included. FIG. 9 illustrates a state in which the support plate 800 is installed inside the mattress 100.

Here, a material of the support plate 800 is not specifically limited, as long as it is strong to easily support the expansion members 120 when the expansion members 120 expand. For example, the material may be plywood, wood, a metal, rubber or the like.

In the human body stretching massage apparatus according to still yet another embodiment of the present invention, a pressure acting upon the expansion of the expansion members 120 is applied toward an upper portion of each of the expansion members 120 by the support plate 800 supporting the expansion members 120 under the expansion members 120 when the expansion members 120 expand, and thus the expansion members 120 may easily expand upon the expansion thereof.

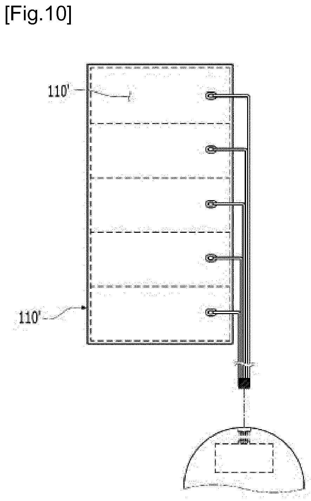

Hereinafter, a human body stretching massage apparatus according to still yet another embodiment of the present invention will be described in detail with reference to FIG. 10, focusing on a difference from the human body stretching massage apparatus according to one embodiment of the present invention. FIG. 10 is a view illustrating a human body stretching massage apparatus according to still yet another embodiment of the present invention.

Referring to FIG. 10, the human body stretching massage apparatus according to still yet another embodiment of the present invention is the same as the human body stretching massage apparatus according to one embodiment of the present invention, except that a mattress 100' is formed by stacking two sheets and an edge of each of the sheets is sewed and an expansion region 100a' is formed to divide the two sheets in a lengthwise direction thereof.

In the human body stretching massage apparatus according to still yet another embodiment of the present invention, since the mattress 100' having a plurality of expansion regions 100a' may be configured with only the two sheets, the human body stretching massage apparatus may be easily manufactured, and a manufacturing cost thereof may be reduced.

The description of the above-described embodiments is provided to enable those skilled in the art to use or implement the present invention. The invention is defined by the claims.

## Claims

1. A human body stretching massage apparatus comprising:
a mattress (100) configured to stretch a human body, including a plurality of expansion regions filled with a filler for expanding the mattress and formed in a rectangular shape;
a filler feeder (200) connected to each of the expansion regions through a connection member (300) to inject the filler into the expansion regions; and
a controller (400) configured to control an operation of the filler feeder (200),
wherein the plurality of expansion regions are arranged parallel in a lengthwise direction of the mattress (100), and each of the expansion region is formed in a rectangular tube shape which accommodates the filler, and
the adjacent expansion regions are partially overlapped with each other when the plurality of expansion regions are arranged parallel in the lengthwise direction of the mattress (100), and
each of the plurality of expansion regions includes a female button (112), and a male button (112') is provided at an inner surface of the mattress, and each of the plurality of expansion regions is fixed to an inside of the mattress by coupling between the female button (112) and the male button (112'), and
the controller (400) includes a control circuit configured with a circuit to control the filler feeder (200), a power supply and operation buttons, and the control circuit includes a timer which counts an injection time to sequentially inject the filler into the plurality of expansion regions for a predetermined time in predetermined order of the plurality of expansion regions, and
the operation buttons include a power button for driving the filler feeder (200), a timer setting button, selection buttons for selecting one or more of the plurality of expansion regions and a selection button for setting the plurality of expansion regions to sequentially expand for a predetermined time, and
the filler feeder (200) is configured to selectively supply the filler into the plurality of expansion regions, and
the filler feeder (200) includes a plurality of electronic valves (330) which open and close the connection members (300) connected to the plurality of expansion regions, and the controller (400) is configured to control opening and closing of the plurality of electronic valves (330), and
by separate expansion and contraction of each of the plurality of expansion regions, each part of the human body is stretched while the human body lies down on the mattress, and
**characterized in that** an acupressure member (710) is formed on a surface of the plurality of expansion regions and comprises a far-infrared lamp (720) in an accommodation space of the acupressure member (710).

2. The apparatus of claim 1, wherein the mattress (100) includes an outer cover (110) and a plurality of expansion regions for forming the expansion regions.

3. The apparatus of claim 2, wherein each of the plurality of expansion regions has a length extending in a direction vertical to a lengthwise direction of the outer cover (110) and the plurality of expansion regions are arranged in the lengthwise direction of the outer cover.

4. The apparatus of claim 3, wherein the plurality of expansion regions are partially overlapped with each other.

5. The apparatus of claim 2, wherein each of the plurality of expansion regions has a length which is equal to or smaller than a half of a width of the outer cover (110) and the plurality of expansion regions are arranged in a lengthwise direction of the outer cover and a direction vertical to the lengthwise direction thereof.

6. The apparatus of claim 3 or 5, wherein each of the plurality of expansion regions is disposed to be spaced apart from each other at a predetermined distance.

7. The apparatus of claim 3, wherein each of the plurality of expansion regions is divided into a plurality of regions by at least one dividing line (121).

8. The apparatus of claim 1, wherein the mattress (100) is formed by stacking two sheets and an edge of each of the sheets is sewed, and the expansion regions are formed to divide the two sheets in a lengthwise direction thereof.

9. The apparatus of claim 1, wherein the apparatus includes a further acupressure member installed inside the mattress.

10. The apparatus of claim 2, further comprising a support plate (800) installed under the mattress or inside the mattress to support the expansion regions when the expansion regions expand.

## Patentansprüche

1. Dehnungsmassagevorrichtung für den menschlichen Körper umfassend:
eine Matratze (100), die dazu konfiguriert ist, einen menschlichen Körper zu dehnen, umfassend eine Vielzahl von Expansionsbereichen, die mit einem Füllstoff zum Expandieren der Matratze gefüllt und in einer rechteckigen Form gebildet sind;
eine Füllstoffzuführeinrichtung (200), die durch ein Verbindungselement (300) mit jedem der Expansionsbereiche verbunden ist, um den Füllstoff in die Expansionsbereiche zu injizieren; und
eine Steuereinrichtung (400), die dazu konfiguriert ist, einen Betrieb der Füllstoffzuführeinrichtung (200) zu steuern,
wobei die Vielzahl von Expansionsbereichen parallel in einer Längsrichtung der Matratze (100) angeordnet sind, und jeder der Expansionsbereiche in Form eines rechteckigen Rohrs gebildet ist, das den Füllstoff aufnimmt, und
die angrenzenden Expansionsbereiche teilweise miteinander überlappt sind, wenn die Vielzahl von Expansionsbereichen parallel in der Längsrichtung der Matratze (100) angeordnet sind, und
jeder der Vielzahl von Expansionsbereichen einen weiblichen Knopf (112) umfasst, und ein männlicher Knopf (112') an einer inneren Oberfläche der Matratze vorgesehen ist, und jeder der Vielzahl von Expansionsbereichen durch Koppeln zwischen dem weiblichen Knopf (112) und dem männlichen Knopf (112' ) an einer Innenseite der Matratze fixiert ist, und
die Steuereinrichtung (400) eine Steuerschaltung umfasst, die mit einer Schaltung zum Steuern der Füllstoffzuführeinrichtung (200), einer Energieversorgung und Betätigungsknöpfen konfiguriert ist, und die Steuerschaltung einen Timer umfasst, der eine Injektionszeit zählt, um den Füllstoff für eine vorgegebene Zeit in einer vorgegebenen Reihenfolge der Vielzahl von Expansionsbereichen sequentiell in die Vielzahl von Expansionsbereichen zu injizieren, und
die Betätigungsknöpfe eine Power-Taste zum Antreiben der Füllstoffzuführeinrichtung (200), einen Timer-Einstellknopf, Auswahlknöpfe zum Auswählen eines oder mehrerer der Vielzahl von Expansionsbereichen und einen Auswahlknopf zum Einstellen der Vielzahl von Expansionsbereichen zum sequentiellen Expandieren für eine vorgegebene Zeit umfassen, und
die Füllstoffzuführeinrichtung (200) dazu konfiguriert ist, den Füllstoff selektiv in die Vielzahl von Expansionsbereichen zuzuführen, und
die Füllstoffzuführeinrichtung (200) eine Vielzahl von elektronischen Ventilen (330) umfasst, die die mit der Vielzahl von Expansionsbereichen verbundenen Verbindungselemente (300) öffnen und schließen, und die Steuereinrichtung (400) dazu konfiguriert ist, ein Öffnen und Schließen der Vielzahl von elektronischen Ventilen (330) zu steuern, und
durch eine separate Expansion und Kontraktion von jedem der Vielzahl von Expansionsbereichen jeder Teil des menschlichen Körpers gedehnt wird, während der menschliche Körper auf der Matratze liegt, und
**dadurch gekennzeichnet, dass**
ein Akupressur-Element (710) auf einer Oberfläche der Vielzahl von Expansionsbereichen gebildet ist und eine Ferninfrarotlampe (720) in einem Aufnahmeraum des Akupressur-Elements (710) umfasst.

2. Vorrichtung nach Anspruch 1, wobei die Matratze (100) eine äußere Abdeckung (110) und eine Vielzahl von Expansionsbereichen zum Bilden der Expansionsbereiche umfasst.

3. Vorrichtung nach Anspruch 2, wobei jeder der Vielzahl von Expansionsbereichen eine Länge aufweist, die sich in einer zu einer Längsrichtung der äußeren Abdeckung (110) vertikalen Richtung erstreckt und die Vielzahl von Expansionsbereichen in der Längsrichtung der äußeren Abdeckung angeordnet sind.

4. Vorrichtung nach Anspruch 3, wobei die Vielzahl von Expansionsbereichen teilweise miteinander überlappt sind.

5. Vorrichtung nach Anspruch 2, wobei jeder der Vielzahl von Expansionsbereichen eine Länge aufweist, die gleich einer Hälfte einer Breite der äußeren Abdeckung (110) oder kleiner als diese ist und die Vielzahl von Expansionsbereichen in einer Längsrichtung der äußeren Abdeckung und einer zu der Längsrichtung von dieser vertikalen Richtung angeordnet sind.

6. Vorrichtung nach Anspruch 3 oder 5, wobei jeder der Vielzahl von Expansionsbereichen dazu angeordnet ist, voneinander in einer vorgegebenen Distanz beabstandet zu sein.

7. Vorrichtung nach Anspruch 3, wobei jeder der Vielzahl von Expansionsbereichen durch wenigstens eine Trennlinie (121) in eine Vielzahl von Bereichen geteilt ist.

8. Vorrichtung nach Anspruch 1, wobei die Matratze (100) durch Stapeln von zwei Lagen gebildet wird und eine Kante von jeder der Lagen genäht ist, und die Expansionsbereiche dazu gebildet sind, die zwei Lagen in einer Längsrichtung von diesen zu teilen.

9. Vorrichtung nach Anspruch 1, wobei die Vorrichtung ein weiteres Akupressur-Element umfasst, das innen in der Matratze installiert ist.

10. Vorrichtung nach Anspruch 2, ferner umfassend eine Trägerplatte (800), die unter der Matratze oder innen in der Matratze installiert ist, um die Expansionsbereiche zu tragen, wenn die Expansionsbereiche expandieren.

## Revendications

1. Appareil de massage par étirement du corps humain comprenant :
un matelas (100) configuré pour étirer un corps humain, comprenant une pluralité de régions de dilatation remplies d'une matière de remplissage pour dilater le matelas et de forme rectangulaire ;
un dispositif d'alimentation de la matière de remplissage (200) connecté à chacune des régions de dilatation via un élément de raccordement (300) pour injecter la matière de remplissage dans les régions de dilatation ; et
un dispositif de commande (400) configuré pour commander un fonctionnement du dispositif d'alimentation de la matière de remplissage (200),
la pluralité des régions de dilatation étant agencées parallèlement dans une direction longitudinale du matelas (100), et chacune des régions de dilatation étant en forme de tube rectangulaire qui reçoit la matière de remplissage, et
les régions de dilatation adjacentes se chevauchant partiellement les unes les autres lorsque la pluralité de régions de dilatation sont agencées parallèlement dans la direction longitudinale du matelas (100), et
chacune des régions de dilatation de la pluralité de régions de dilatation comprenant un bouton femelle (112), et un bouton mâle (112') étant prévu sur une surface intérieure du matelas, et chacune des régions de dilatation de la pluralité de régions de dilatation étant fixée à un intérieur du matelas par couplage entre le bouton femelle (112) et le bouton mâle (112'), et
le dispositif de commande (400) comprenant un circuit de commande configuré avec un circuit pour commander le dispositif d'alimentation de la matière de remplissage (200), une alimentation électrique et des boutons d'actionnement, et le circuit de commande comprenant une minuterie qui compte une durée d'injection pour injecter de façon séquentielle la matière de remplissage dans la pluralité de régions de dilatation pendant un temps prédéterminé dans un ordre prédéterminé de la pluralité de régions de dilatation, et
les boutons d'actionnement comprenant un bouton d'alimentation pour commander le dispositif d'alimentation de la matière de remplissage (200), un bouton de réglage de la minuterie, des boutons de sélection pour sélectionner une ou plusieurs régions de dilatation de la pluralité de régions de dilatation, et un bouton de sélection pour régler la pluralité de régions de dilatation afin qu'elles se dilatent de façon séquentielle pendant une durée prédéterminée, et
le dispositif d'alimentation de la matière de remplissage (200) étant configuré pour alimenter sélectivement la matière de remplissage dans la pluralité de régions de dilatation, et
le dispositif d'alimentation de la matière de remplissage (200) comprenant une pluralité de vannes électroniques (330) qui ouvrent et ferment les éléments de raccordement (300) connectés à la pluralité de régions de dilatation, et le dispositif de commande (400) étant configuré pour commander l'ouverture et la fermeture de la pluralité de vannes électroniques (330), et
chaque partie du corps humain étant étirée, alors que le corps humain est couché sur le matelas, par dilatation et contraction séparées de chacune des régions de dilatation de la pluralité de régions de dilatation, et
**caractérisé en ce qu'**un élément d'acupression (710) est formé sur une surface de la pluralité de régions de dilatation et comprend une lampe à infrarouge lointain (720) dans un espace de logement de l'élément d'acupression (710).

2. Appareil selon la revendication 1, dans lequel le matelas (100) comprend une couverture extérieure (110) et une pluralité de régions de dilatation pour former les régions de dilatation.

3. Appareil selon la revendication 2, dans lequel chacune des régions de dilatation de la pluralité de régions de dilatation a une longueur s'étendant dans une direction verticale par rapport à une direction longitudinale de la couverture extérieure (110) et la pluralité de régions de dilatation est agencée dans la direction longitudinale de la couverture extérieure.

4. Appareil selon la revendication 3, dans lequel la pluralité de régions de dilatation se chevauchent partiellement les unes les autres.

5. Appareil selon la revendication 2, dans lequel chacune des régions de dilatation de la pluralité de régions de dilatation a une longueur qui est égale ou inférieure à la moitié d'une largeur de la couverture extérieure (110), et la pluralité de régions de dilatation sont agencées dans une direction longitudinale de la couverture extérieure et dans une direction verticale par rapport à la direction longitudinale de celle-ci.

6. Appareil selon la revendication 3 ou 5, dans lequel chacune des régions de dilatation de la pluralité de régions de dilatation est disposée de manière à être espacée les unes des autres à une distance prédéterminée.

7. Appareil selon la revendication 3, dans lequel chacune des régions de dilatation de la pluralité de régions de dilatation est divisée en une pluralité de régions par au moins une ligne de division (121).

8. Appareil selon la revendication 1, dans lequel le matelas (100) est formé par l'empilage de deux feuilles et un bord de chacune des feuilles est cousu, et les régions de dilatation sont formées pour diviser les deux feuilles dans une direction longitudinale de celles-ci.

9. Appareil selon la revendication 1, dans lequel l'appareil comprend un élément d'acupression supplémentaire installé à l'intérieur du matelas.

10. Appareil selon la revendication 2, comprenant en outre une plaque de soutien (800) installée sous le matelas ou à l'intérieur du matelas pour soutenir les régions de dilatation lorsque les régions de dilatation se dilatent.
